# EUROPÄISCHE PATENTSCHRIFT

(11) **EP 2 214 562 B1**
(45) Veröffentlichungstag und Bekanntmachung des Hinweises auf die Patenterteilung: **27.04.2016**
(21) Anmeldenummer: 08847587.6
(22) Anmeldetag: 04.11.2008
(51) Int. Cl.: A61B 17/00, A61B 17/12, A61B 18/14, A61B 17/28, A61B 17/285, A61B 17/32, A61B 18/00, A61B 90/00

(54) **CHIRURGIEGERÄT ZUM VERSCHLIESSEN VON BLUTGEFÄSSEN UND WÄRMEAUSHÄRTBARER KLEBSTOFF ALS ARZNEIMITTEL**
SURGICAL INSTRUMENT FOR SEALING BLOOD VESSELS, AND HEAT-CURABLE ADHESIVE AS A MEDICAMENT
APPAREIL CHIRURGICAL POUR SCELLER DES VAISSEAUX SANGUINS ET ADHÉSIF THERMODURCISSABLE SERVANT D'AGENT PHARMACEUTIQUE

(30) Priorität: 05.11.2007 DE 102007052641; 27.12.2007 DE 102007062786
(43) Veröffentlichungstag der Anmeldung: 11.08.2010
(73) Patentinhaber: ERBE Elektromedizin GmbH, 72072 Tübingen (DE)
(72) Erfinder: SCHÄLLER, Daniel, 72074 Tübingen (DE); VOIGTLÄNDER, Matthias, 72810 Gomaringen (DE); FISCHER, Klaus, 72202 Nagold (DE); SZYRACH, Mara, 8052 Zürich (CH); BLOBEL, Lars, 72119 Ammerbruch-Entringen (DE); SIGLE, Irina, 72116 Mössingen (DE)
(74) Vertreter: Bohnenberger, Johannes
(86) Internationale Anmeldenummer: PCT/EP2008/009289
(87) Internationale Veröffentlichungsnummer: WO 2009/059741

(56) Entgegenhaltungen:
- WO-A-2005/082299
- WO-A-2007/058877
- US-A- 5 725 568
- US-A- 6 033 427
- US-A1- 2001 016 750
- US-A1- 2002 099 372
- US-A1- 2002 165 593
- US-A1- 2003 097 149
- US-A1- 2006 167 452

## Beschreibung

Die Erfindung betrifft ein Chirurgiegerät zum Verschließen von Blutgefäßen.

In der Chirurgie müssen oftmals Blutgefäße abgetrennt und nach dem Abtrennen verschlossen werden, um eine Blutung zu vermeiden. Ein solcher Verschluss von Blutgefäßen wird herkömmlicherweise mit einer so genannten Fadenligatur vorgenommen, bei welcher beide Enden des Gefäßes mit einem Faden abgebunden werden, bevor das Blutgefäß dazwischen getrennt wird.

Bei neueren Verfahren wird eine Hochfrequenz-Koagulation mit chirurgischen Zangen oder Pinzetten vorgenommen, bei welcher mit zwei einander gegenüber liegenden Flächenelektroden eine Mindestanpresskraft auf die Gefäßwände ausgeübt wird, während der HF-Energieeintrag über die Flächenelektroden für eine Erwärmung des Gefäßmaterials sorgt. Damit werden eine Koagulation und ein Verbund der einander gegenüber liegenden Gefäßwände bewerkstelligt, das Gefäß wird sozusagen versiegelt. Derartige Koagulationsgeräte sind beispielsweise aus der DE 42 42 143 C2 oder der EP 0 997 108 A2 bekannt.

Aus der US 6 033 427 A ist ein Chirurgiegerät nach dem Oberbegriff von Anspruch 1 bekannt.

Während kleinere Gefäße (unter 1 mm Durchmesser) sich durch den ihnen eigenen Schrumpfungsprozess verschließen, ist das Verschließen größerer Gefäße problematisch, da die Verschlussstellen auch dem maximalen Gefäßinnendruck des Kreislaufsystems standhalten müssen. Dieses Problem steigt mit der Größe (dem Durchmesser) des Blutgefäßes.

Der Erfindung liegt die Aufgabe zu Grunde, eine Vorrichtung aufzuzeigen, mittels derer ein sicherer Verschluss von Blutgefäßen in einfacher Weise bewerkstelligt werden kann.

Diese Aufgabe wird durch ein Chirurgiegerät nach Anspruch 1 gelöst.

Ein wesentlicher Punkt der Erfindung liegt darin, dass zum Verschließen eines Blutgefäßes in dieses ein Klebstoff injiziert wird, so dass das Blutgefäß nach Aushärtung des Klebstoffes abgetrennt werden kann. Ein kompliziertes Hantieren mit dem Klebstoff entfällt somit.

Mit dem Begriff "Klebstoff" ist hier ganz allgemein eine Wirksubstanz gemeint, die dazu geeignet ist, die Oberflächen miteinander zu verbinden. Dies kann also ein Klebstoff im technischen Sinne ebenso sein, wie z. B. ein Wirkstoff, der über eine enzymatische Reaktion (also indirekt) diese Verbindung bewirkt.

Genauer gesagt, wird die Aufgabe durch ein Chirurgiegerät zum Verschließen von Blutgefäßen gelöst, welches eine Injektionseinrichtung und eine Dosiereinrichtung umfasst, die derart aufgebaut sind, dass eine vorbestimmte Menge eines Klebstoffes oder eines Klebstoffes (bzw. Wirkstoffes) mit Füllstoff in einen Verschlussabschnitt des Blutgefäßes injizierbar ist.

Eine weitere Besonderheit liegt hierbei darin, dass ein definierter Verschlussabschnitt des Blutgefäßes mit Klebstoff gefüllt wird, der Klebstoff also nicht im Blutgefäß weiter wandert.

Vorzugsweise wird weiterhin eine Klammereinrichtung vorgesehen, die derart ausgebildete Greifelemente aufweist, dass das Blutgefäß an voneinander beabstandeten Verschlussstellen derart greif- und verschließbar ist, dass zwischen den Verschlussstellen der mit dem Klebstoff befüllbare Verschlussabschnitt des Blutgefäßes verbleibt. Dadurch wird in einfacher Weise ein Weiterwandern des Klebstoffes in das Blutgefäß verhindert.

Die Injektionseinrichtung ist vorzugsweise derart mit der Klammereinrichtung verbunden, dass der Klebstoff in den Verschlussabschnitt injizierbar ist. Die Klammereinrichtung und die Injektionseinrichtung sind also in ihren räumlichen Positionen zueinander definiert, so dass ein optisch gesteuertes Zielen und kompliziertes Hantieren entfällt. Vorzugsweise weist die Injektionseinrichtung eine Düse auf, die insbesondere bewegbar ist und umfasst eine Druckerzeugungseinrichtung, die derart ausgebildet ist, dass der Klebstoff unter Bildung eines hinreichend starken Strahls in das Blutgefäß injizierbar ist. Es wird in diesem Fall also keine Injektionsnadel verwendet, was dazu führt, dass der Aufbau der chirurgischen Vorrichtung sehr einfach ist.

Alternativ kann die Injektionseinrichtung eine vorzugsweise bewegbare Nadel und eine Druckerzeugungseinrichtung umfassen, die derart ausgebildet sind, dass der Klebstoff mittels der Nadel in das Blutgefäß injizierbar ist. Besonders bei dieser Ausführungsform können Einweg-Patronen, umfassend die Injektionsnadel, einen Kolben und einen darin befindlichen Zylinder verwendet werden, was Sterilitätsprobleme sicher vermeidet. Hierbei kann die Injektionseinrichtung derart ausgebildet sein, dass die Nadel durch die Druckerzeugungseinrichtung insbesondere hydraulisch bewegbar ist. Es sind in diesem Fall also keine weiteren mechanischen Teile notwendig.

Vorzugsweise ist eine Energiezufuhreinrichtung zum Aufheizen und/oder Koagulieren des Verschlussabschnittes vorgesehen. Wenn der Verschlussabschnitt bzw. die dort befindlichen Gefäßabschnitte-koaguliert werden, so wird ein besonders haltbarer Verschluss erzielt. Darüber hinaus aber kann durch Aufheizen oder auch eine andere Form von Energiezufuhr ein Aushärten des Klebstoffes bewerkstelligt werden, wenn ein entsprechender Klebstoff, der in diesem Fall als Arzneimittel fungiert, verwendet wird. Insbesondere dann, wenn der Klebstoff zusätzlich noch mit Füllstoffen versehen ist, kann in einfacher Weise auch ein großer Querschnitt eines Gefäßes versiegelt werden.

Vorzugsweise umfasst die Energiezufuhreinrichtung Elektroden zwischen den Greifelementen zum Durchleiten eines HF-Stromes durch das Blutgefäß und zwar begrenzt auf dem Verschlussabschnitt. Zum Koagulieren ist dies an sich bekannt. Wenn der klebstoff elektrisch leitend ist und/oder mit elektrisch leitenden Füllstoffen versehen ist, so kann auf diese Weise besonders einfach eine Erwärmung zum Aushärten des Klebstoffes bewerkstelligt werden.

Vorzugsweise ist eine Schneideinrichtung vorgesehen, zum Durchtrennen des Blutgefäßes im Bereich des Verschlussabschnittes. Auf diese Weise kann mit ein und demselben Chirurgiegerät in zeitsparender Weise die Durchtrennung des Gefäßes geschehen.

Vorzugsweise ist eine erste Messeinrichtung zum Feststellen eines Befüllungszustandes des Verschlussabschnittes vorgesehen. Dadurch ist es möglich, die Menge an Klebstoff, die in den Verschlussabschnitt eingespritzt wird, exakt zu kontrollieren. Diese Messeinrichtung ist vorzugsweise über eine erste Steuerung zur Steuerung eines Injektionszeitpunktes und/oder eines -verlaufes und/oder einer Injektionsmenge mit der Injektionseinrichtung verbunden. Es entfällt damit eine nur erfahrenen Chirurgen mögliche Steuerung des Befüllungsvorganges.

Vorzugsweise ist auch eine zweite Messeinrichtung zum Feststellen eines Temperatur- und/oder Koagulationszustandes des Verschlussabschnittes vorgesehen, so dass eine vollständige Kontrolle über den Operationsvorgang möglich ist. Vorzugsweise ist die zweite Messeinrichtung über eine zweite Steuerung mit einer Energiezufuhreinrichtung zum Zuführen von Energie zum Verschlussabschnitt zur Steuerung der Energiemenge verbunden, so dass hier relativ wenigen, erfahrenen Operateuren die Benutzung des Chirurgiegerätes ermöglicht wird.

Die erste und/oder die zweite Messeinrichtung sind vorzugsweise als Impedanzmesseinrichtungen zum Messen einer elektrischen Impedanz des Blutgefäßes, insbesondere im Bereich des Verschlussabschnittes ausgebildet.

Dieser Messparameter gibt in relativ einfacher Weise genaue Messdaten wieder, die den Operationsverlauf überwachbar machen. Vorzugsweise wird diese Impedanzmesseinrichtung unter gleichzeitiger Verwendung der zum Koagulieren und/oder Aufheizen des Verschlussabschnittes vorgesehenen Elektroden kombiniert, so dass die Elektroden sowohl als Koagulationselektroden, als auch als Messelektroden einsetzbar sind. In diesem Fall erfolgt vorzugsweise eine Umschaltung zwischen der Energiequelle (Hochfrequenzgenerator) und der Messeinrichtung (Impedanzmesseinrichtung) in relativ kurzen Zeitabschnitten, so dass der Verlauf des Koagulations- bzw. Aufheizungsvorganges exakt bestimmbar ist.

Wie oben bereits angedeutet, ist bei der Erfindung ein wärmeaushärtbarer Klebstoff als Arzneimittel in der beschriebenen Weise anwendbar. Insbesondere zum Verschließen von Blutgefäßen eignet sich dieses Arzneimittel hervorragend und umfasst vorzugsweise einen Füllstoff oder eine Füllstoffmischung, so dass auch größere Querschnitte von Gefäßen sicher verschließbar sind.

Der Klebstoff weist vorzugsweise eine derart erhöhte Leitfähigkeit auf, dass er durch Durchleiten eines elektrischen Stroms, insbesondere eines hochfrequenten Stroms, erwärmbar ist, so dass eine Aushärtung in kontrollierter und einfacher Weise erfolgen kann. Besonders bevorzugt sind hierbei Aushärtetemperaturen, die über der normalen Raumtemperatur oder auch Körpertemperatur liegen, also vorzugsweise über 38° C, vorzugsweise über 42° C und besonders vorzugsweise über 55° C, also bei Temperaturen, in denen Gewebe bereits zu koagulieren beginnt. Auf diese Weise ist sichergestellt, dass der Klebstoff nur dort aushärtet, wo er aushärten soll, nämlich im Verschlussabschnitt. Der Klebstoff ist biokompatibel und vorzugsweise derart ausgebildet, dass ein Abbau unausgehärteter Klebstoffanteile im Körper erfolgen kann.

Nachfolgend werden bevorzugte Ausführungsformen der Erfindung anhand von Abbildungen näher erläutert. Hierbei zeigen
- - Fig. 1: ein Chirurgiegerät bzw. -instrument in einer Seitenansicht;
- - Fig. 2 u. Fig. 3: perspektivische Ansichten von Endabschnitten des Instruments nach Fig. 1;
- - Fig. 4: einen Schnitt entlang der Linie IV-IV aus Fig. 1;
- - Fig. 5: eine Ansicht der Anordnung nach Fig. 4, jedoch mit einem "eingeklemmten" Gefäß;
- - Fig. 6: einen Schnitt entlang der Linie VI-VI aus Fig. 5;
- - Fig. 7: eine Teil-Darstellung zur Erläuterung einer ersten Ausführungsform einer Injektionsvorrichtung;
- - Fig. 8: eine Detail-Darstellung eines Teils der Injektionsvorrichtung nach Fig. 7;
- - Fig. 9: eine weitere Ausführungsform einer Injektionsvorrichtung in einer Darstellung ähnlich der nach Fig. 7;
- - Fig. 10: eine Schnittdarstellung in einer Ansicht ähnlich der nach Fig. 4, jedoch durch eine weitere Ausführungsform der Erfindung;
- - Fig. 11: eine perspektivische Teilansicht einer Schneideinrichtung;
- - Fig. 12: eine weitere Ausführungsform eines Instrumentes in einer Seitenansicht ähnlich der nach Fig. 1;
- - Fig. 13: einen Schnitt entlang der Linie XIII-XIII aus Fig. 12;
- - Fig. 14: einen Schnitt entlang der Linie XIV-XIV aus Fig. 12;
- - Fig. 15: schematisierter Gesamtaufbau des Chirurgiegerätes mit peripheren Einrichtungen; und
- - Fig. 16: ein Flussdiagramm zur Erläuterung der Bedienung des Chirurgiegerätes.

In der nachfolgenden Beschreibung werden für gleiche und gleich wirkende Teile dieselben Bezugsziffern verwendet.

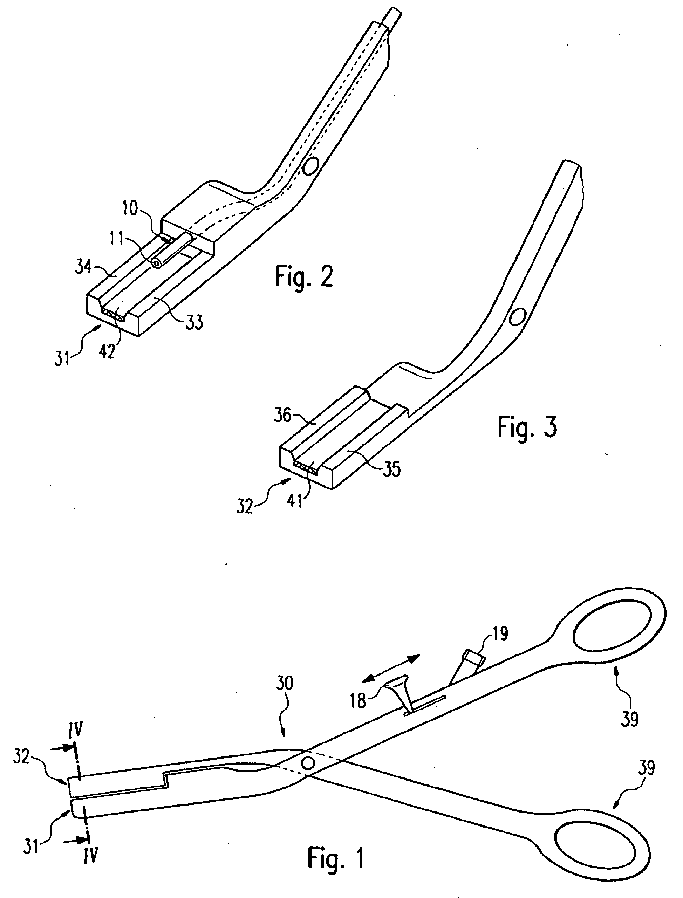

In Fig. 1 ist in einer Draufsicht eine scherenartige Ausführungsform der erfindungsgemäßen Klammereinrichtung gezeigt. Diese umfasst Griffabschnitte 39, die über ein Gelenk miteinander verbunden sind. Den Griffabschnitten bezüglich des Gelenks gegenüber liegend sind Greifabschnitte 31, 32 vorgesehen, mit denen ein Gefäß ergriffen werden kann. Weiterhin ist an einer Branche der Griffabschnitte 39 ein Betätigungshebel zum Betätigen einer Injektionseinrichtung - diese wird weiter unten beschrieben - vorgesehen. Mit der Bezugsziffer 19 ist eine Flüssigkeitszufuhreinrichtung bezeichnet.

Die Fig. 2 und 3 zeigen perspektivische Draufsichten auf die Greifabschnitte 31 und 32. Wie aus den Abbildungen hervorgeht, sind die Greifabschnitte 31, 32 muldenförmig ausgebildet und umfassen jeweils Greifelemente 33, 34 bzw. 35, 36 und zwischen diesen tiefer liegende Elektroden 41 bzw. 42, die gegenüber den Greifelementen 33-36 isoliert angebracht sind. Die Elektroden 41, 42 sind über (nicht gezeigte) elektrische Verbindungselemente mit einem Hochfrequenzgenerator verbindbar.

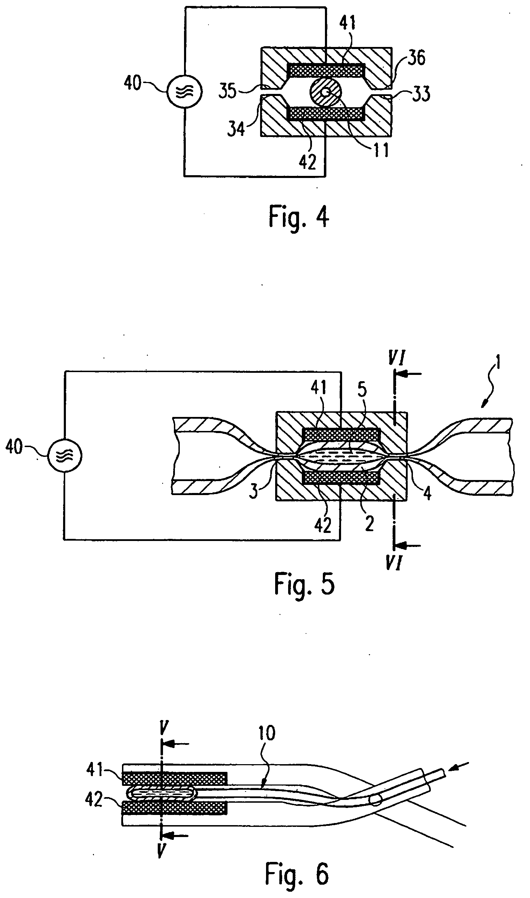

Der Greifabschnitt 31 weist weiterhin eine Injektionseinrichtung 10 mit einer Düse 11 auf, deren Funktion nachfolgend erläutert wird. Die Düse 11 ist hierbei derart symmetrisch platziert, wie dies in Fig. 4 gezeigt ist. Sie sitzt also im Wesentlichen mittig zwischen den beiden Elektroden 41, 42.

Wird mit der Klammereinrichtung 30 ein Gefäß 1 ergriffen, wie bei den Fig. 5 und 6 (in zwei verschiedenen Schnittebenen) gezeigt ist, so werden die Gefäßwände durch die Greifelemente 33-36 zusammengedrückt, so dass Verschlussstellen 3, 4 gebildet werden, an denen das Gefäß 1 vollständig dicht verschlossen ist. Zwischen den Verschlussstellen 3, 4 wird dadurch, dass die Greifabschnitte 31, 32 wannenförmig ausgebildet sind, die den jeweiligen Wannenboden bildenden Elektroden 41, 42 gegenüber den Greifelementen 33-36 also zurückversetzt sind, ein Hohlraum gebildet, in welchem ein Verschlussabschnitt des Gefäßes 1 gebildet ist, der an beiden Seiten durch die Verschlussstellen 3, 4 begrenzt ist.

In diesen Verschlussabschnitt 2 wird nun mittels der Düse 11 ein Klebstoff injiziert, der vorzugsweise mit einem Füller versehen ist und den Verschlussabschnitt 2 nach der Injektion ausfüllt, wie dies in Fig. 5 gezeigt ist. Diese Injektion des Klebstoffes erfolgt bei der hier gezeigten Ausführungsform über einen erhöhten Druck (ähnlich den aus der Wasserstrahlchirurgie bekannten Geräten), wozu die Injektionseinrichtung 10 wie in Fig. 6 gezeigt ist, mit ihrer Düse 11 im Wesentlichen direkt auf die Außenwand des Blutgefäßes 1 im Bereich des Verschlussabschnittes 2 aufgesetzt wird.

Der Klebstoff ist vorzugsweise derart ausgebildet, dass er bei einer erhöhten Temperatur oder auch durch ein Hindurchleiten von elektrischem Strom direkt aushärtet. Weiterhin wird durch das Hindurchleiten eines HF-Stroms das Blutgefäß 1 im Bereich des Verschlussabschnittes 2 koaguliert, also devitalisiert, wenn dies gewünscht ist. Dies wird in den Fig. 4 und 5 gezeigten HF-Generatoren 40 angedeutet.

Sobald der Kleber ausgehärtet ist, bildet er einen mit den Wänden des Blutgefäßes 1 klebend verbundenen Pfropfen. Dieser kann nun durchtrennt werden, so dass das Blutgefäß 1 sicher verschlossen ist.

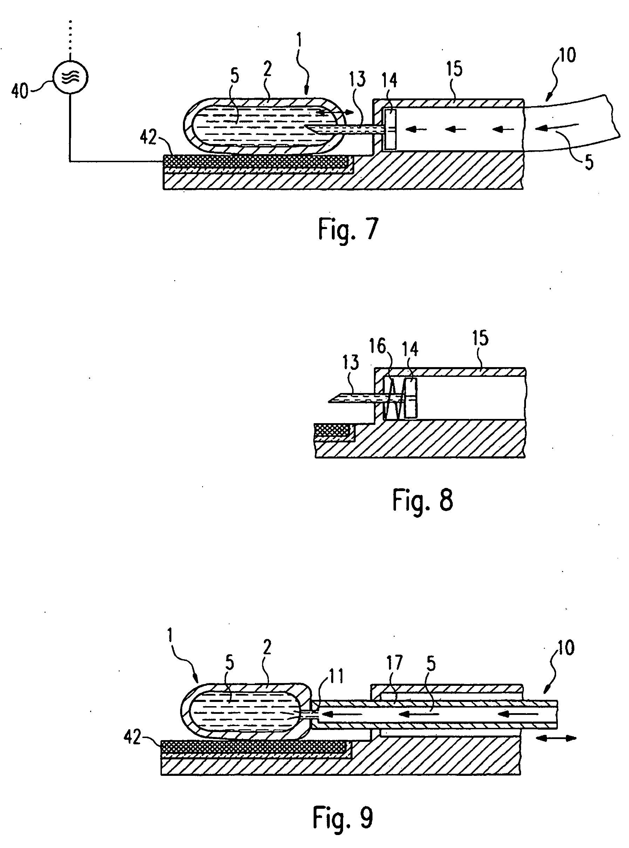

Bei der in Fig. 7 gezeigten Variante wird der Klebstoff 5 in den Verschlussabschnitt 2 mittels einer Injektionsnadel 3 injiziert. Diese Injektionsnadel 3 sitzt an ihrem, der Spitze gegenüber liegenden Ende in einem Kolben 14, der wiederum in einem Zylinder 15 bewegbar ist. Im Ruhezustand nimmt die Injektionsnadel 13 eine zurückgezogene Position ein, so dass ihre Spitze aus dem Zylinder 15 nicht hervorsteht. Dies kann - wie in Fig. 8 gezeigt - durch eine Rückholfeder 16 bewerkstelligt werden. Bei diesen, in den Fig. 7 und 8 gezeigten Ausführungsformen wird also durch das Injizieren des Klebstoffes selbst auch die Injektionsnadel 13 bewegt.

Bei der in Fig. 9 gezeigten Ausführungsform wird die Injektion durch einen erhöhten Druck bewirkt (ähnlich wie in der Wasserstrahlchirurgie). Der Schlauch 17, in welchem die Düse 11 sitzt, wird bei dieser Ausführungsform der Erfindung ebenfalls bewegt und zur Injektion des Klebstoffes auf das Blutgefäß 1 bzw. den Verschlussabschnitt 2 aufgesetzt.

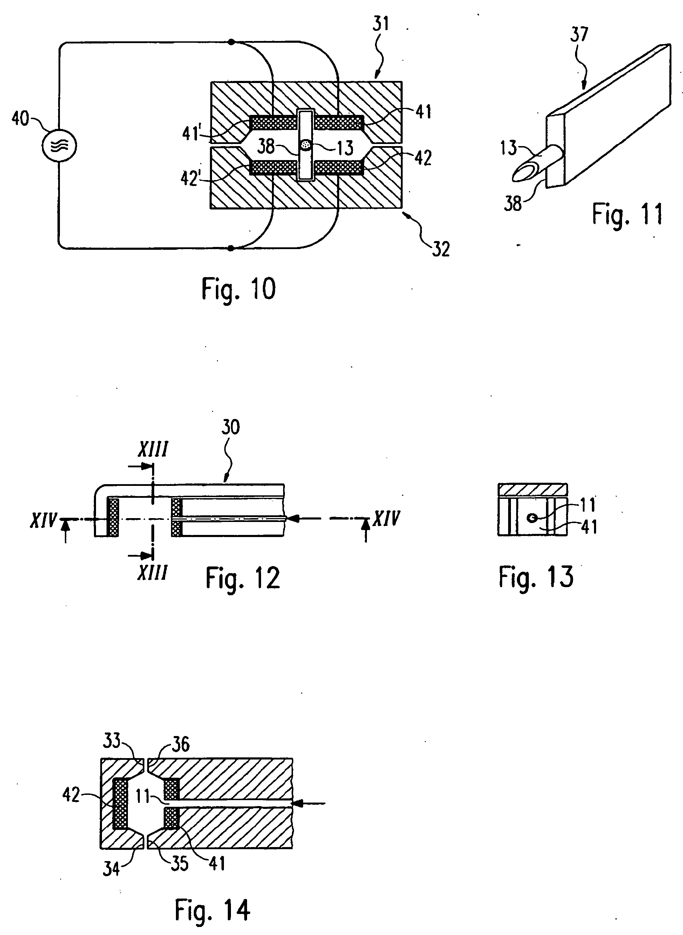

Bei der in den Fig. 7 und 11 gezeigten Ausführungsform der Erfindung ist zusätzlich noch eine Schneideinrichtung 37 vorgesehen. An deren Vorderende befindet sich eine Schneide 38, die nach Aushärten des Klebstoffes das Blutgefäß 1 im Bereich des Verschlussabschnittes 2 durchtrennen kann. Weiterhin ist die Injektionsnadel 13 in der Schneidvorrichtung 37 angebracht, so dass ein äußerst kompakter Aufbau entsteht.

Die Elektroden sind bei dieser Ausführungsform der Erfindung in Teilabschnitte 41, 41' bzw. 42, 42' unterteilt, damit die Schneideinrichtung 37 in den Greifabschnitten 31, 32 geführt laufen kann. Auch bei dieser Ausführungsform ist es möglich, an Stelle einer Nadel 13 mit einer Düse 11 zu arbeiten, durch welche Klebstoff bei erhöhtem Druck in das Blutgefäß 1 injiziert wird.

Die Ausführungsform nach den Fig. 12-14 zeigt den distalen Abschnitt einer anders ausgebildeten Klammereinrichtung 30, bei der keine zwei Branchen über ein Gelenk verbunden, sondern eine schiebebewegliche Anordnung getroffen ist. Bei dieser Anordnung sitzt die Düse 11 direkt in der Fläche der einen Elektrode 41. Selbstverständlich ist auch hier die Benutzung einer Nadel 13 möglich.

Bei den zuvor gezeigten Ausführungsformen, insbesondere bei der Ausführungsform nach den Fig. 7-9 sind Injektionseinrichtungen 10 vorgesehen, welche über einen Schlauch mit einer entsprechenden Pumpeinrichtung verbunden sind. Es ist aber in allen Fällen auch möglich, Einweg-Behälter vorzusehen, in denen eine zumindest hinreichende Klebstoffmenge gelagert ist. Diese Einweg-Behälter können entweder mit einer Nadel 13 oder aber einer entsprechenden Düse 11 versehen sein, was Sterilitätsanforderungen leicht erfüllbar macht.

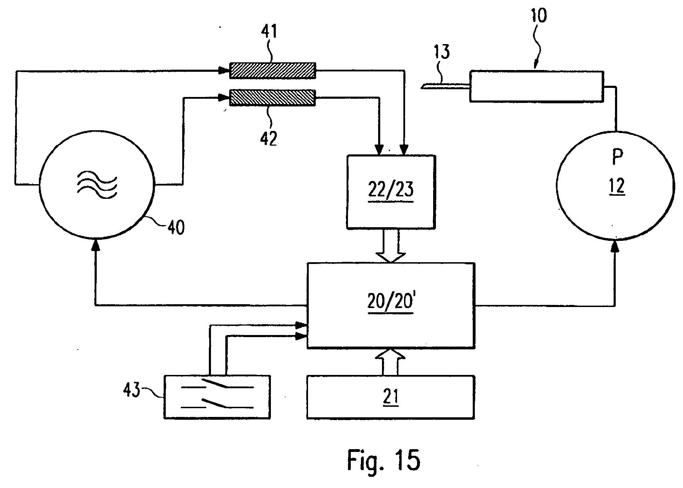

In Fig. 15 ist schematisiert ein Gesamtaufbau gezeigt, der zur Benutzung des erfindungsgemäßen Chirurgiegerätes dient.

Die Elektroden sind wieder mit den Bezugsziffern 41, 42 eingezeichnet und sind an den Generator 40 angeschlossen, der von einer Steuereinrichtung 20, 20' angesteuert wird. Weiterhin sind die Elektroden 41, 42 mit einer Messeinrichtung 22, 23 verbunden, die derart ausgebildet ist, dass die Impedanz zwischen den Elektroden 41, 42, also die Impedanz des Verschlussabschnittes 2 gemessen werden kann. Die Messwerte werden der Steuerung 20, 20' zugeführt.

Die Steuerung 20, 20' steuert weiterhin eine Druckerzeugungseinrichtung 12, welche die Injektionseinrichtung 10 speist, so dass über die hier gezeigte Nadel 13 Flüssigkeit in den Verschlussabschnitt 2 injiziert werden kann.

Weiterhin sind eine Einstelleinrichtung 21 zum Einstellen der verschiedenen Parameter des Generators 40 und der Druckerzeugungseinrichtung 12 sowie eine Schalteinrichtung 43, die als Fuß- oder Fingerschalter ausgebildet sein kann, zur Beeinflussung der Steuerung 20, 20' vorgesehen. Die Funktionsweise der Einzelteile wurde weiter oben bereits beschrieben.

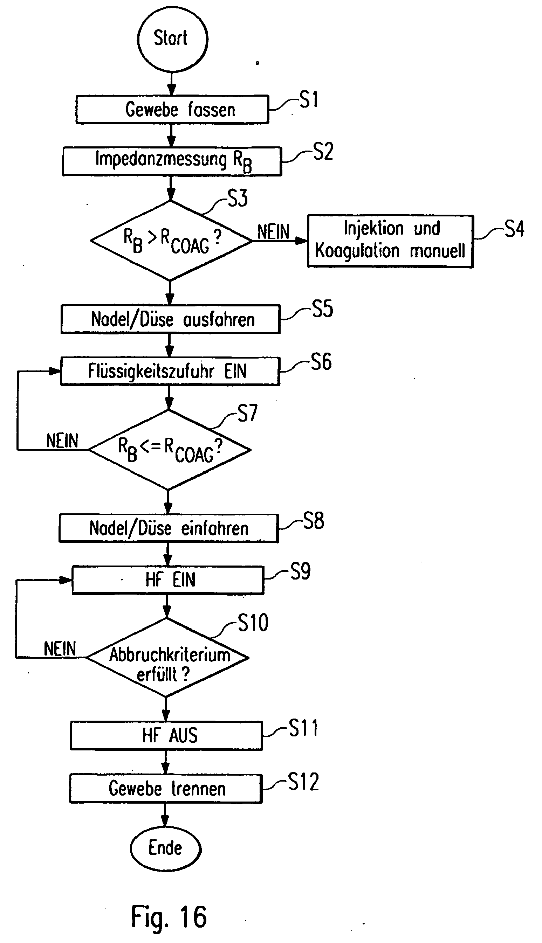

In Fig. 16 ist ein schematisiertes (vereinfachtes) Flussdiagramm gezeigt.

In einem ersten Schritt S1 wird das Gewebe (durch den Operateur) gefasst. Danach folgt in einem Schritt S2 eine Impedanzmessung, in welcher die Messeinrichtung 22/23 die Impedanz zwischen den Elektroden 41, 42 misst.

In einem Schritt S3 wird abgefragt, ob die Impedanz R_{B} größer ist als eine vorbestimmte Impedanz R_{COAG}, also eine Impedanz, die anzeigt, dass die Gewebewand zur aktiven Elektrode ausreichend Kontakt hat. Ist dies nicht der Fall, so kann eine manuelle Injektion und Koagulation erfolgen, der Automatismus wird gestoppt.

Ansonsten wird in einem Schritt S5 die Nadel bzw. die Düse ausgefahren, so dass die Nadel in das Gefäß einsticht bzw. die Düse am Gefäß anliegt.

In einem Schritt S6 wird die Flüssigkeitszufuhr eingeschaltet, es wird also Kleber samt Füller injiziert.

In einem Schritt S7 wird abgefragt, ob der Widerstand R_{B} kleiner oder gleich der vorbestimmten Impedanz R_{COAG} ist. Ist dies nicht der Fall, so wird der Schritt S6 wiederholt, es wird also weitere Flüssigkeit injiziert.
Ist dies aber der Fall, so wird in einem Schritt S8 die Nadel bzw. die Düse eingefahren und danach in einem Schritt S9 der Hochfrequenzstrom eingeschaltet.

In einem Schritt S10 wird ein Abbruchkriterium geprüft, aufgrund dessen entschieden wird, ob das Gewebe hinreichend koaguliert und/oder der Klebstoff hinreichend augehärtet ist. Ist dies nicht der Fall, so wird der Schritt S9 wiederholt.

Ist dies der Fall, so wird in einem Schritt S11 die Energiezufuhr (durch die Höchfrequenz-Quelle öder einer andere Energiequelle) ausgeschaltet.

Danach wird in einem Schritt S12 das Gewebe getrennt. Die beiden Enden des Gefäßes sind nun korrekt verschlossen.

### Bezugszeichenliste

- 1: Blutgefäß
- 2: Verschlussabschnitt
- 3: Verschlussstelle
- 4: Verschlussstelle
- 5: Klebstoff
- 10: Injektionseinrichtung
- 11: Düse
- 12: Druckerzeugungseinrichtung
- 13: Nadel
- 14: Kolben
- 15: Zylinder
- 16: Feder
- 17: Schlauch
- 18: Betätigungshebel
- 19: Flüssigkeitszufuhr
- 20, 20': Steuerung
- 21: Einstelleinrichtung
- 22: erste Messeinrichtung
- 23: zweite Messeinrichtung
- 30: Klammereinrichtung
- 31: Greifabschnitt
- 32: Greifabschnitt
- 33: Greifelement
- 34: Greifelement
- 35: Greifelement
- 36: Greifelement
- 37: Schneideinrichtung
- 38: Schneide
- 39: Griffabschnitt
- 40: HF-Generator
- 41: Elektrode
- 42: Elektrode
- 43: Betätigungsschalter

## Patentansprüche

1. Chirurgiegerät zum Verschließen von Blutgefäßen (1), umfassend eine Injektionseinrichtung (10) und eine Dosiereinrichtung (20), die derart aufgebaut sind, dass eine vorbestimmte Menge eines Klebstoffes (5) oder eines Klebstoffes mit Füllstoff in einen Verschlussabschnitt (2) des Blutgefäßes (1) injizierbar ist,
**gekennzeichnet durch**
eine Klammereinrichtung (30), die derart ausgebildete Greifelemente (33-36) aufweist, dass das Blutgefäß (1) an voneinander beabstandeten Verschlussstellen (3, 4) derart greif- und verschließbar ist, dass zwischen den Verschlussstellen (3, 4) der mit dem Klebstoff (5) befüllbare Verschlussabschnitt (2) des Blutgefäßes (1) verbleibt, und eine Energiezufuhreinrichtung (40-42) zum Aufheizen und/oder Koagulieren des Verschlussabschnittes (2), welche Elektroden (41, 42) zwischen den Greifelementen (33-36) zum Durchleiten eines HF-Stromes **durch** das Blutgefäß (1), begrenzt auf den Verschlussabschnitt (2), aufweist.

2. Chirurgiegerät nach Anspruch 1,
**dadurch gekennzeichnet, dass**
die Injektionseinrichtung (10) derart mit der Klammereinrichtung (30) verbunden ist, dass der Klebstoff (5) in den Verschlussabschnitt (2) injizierbar ist.

3. Chirurgiegerät nach einem der vorhergehenden Ansprüche,
**dadurch gekennzeichnet, dass**
die Injektionseinrichtung (10) eine Düse (11) und eine Druckerzeugungseinrichtung (12) umfasst, die derart ausgebildet sind, dass der Klebstoff (5) unter Bildung eines hinreichend starken Strahles in das Blutgefäß (1) injizierbar ist.

4. Chirurgiegerät nach einem der vorhergehenden Ansprüche,
**dadurch gekennzeichnet, dass**
die Injektionseinrichtung (10) eine Nadel (13) und eine Druckerzeugungseinrichtung (12) umfasst, die derart ausgebildet sind, dass der Klebstoff (5) mittels der Nadel (13) in das Blutgefäß (1) injizierbar ist.

5. Chirurgiegerät nach Anspruch 4,
**dadurch gekennzeichnet, dass**
die Injektionseinrichtung (10) derart ausgebildet ist, dass die Nadel (13) durch die Druckerzeugungseinrichtung (12) hydraulisch bewegbar ist.

6. Chirurgiegerät nach einem der vorhergehenden Ansprüche,
**gekennzeichnet durch**
eine Schneideinrichtung (37) zum Durchtrennen des Blutgefäßes (1) im Bereich des Verschlussabschnittes (2).

7. Chirurgiegerät nach einem der vorhergehenden Ansprüche,
**gekennzeichnet durch**
eine erste Messeinrichtung (22) zum Feststellen eines Befüllungszustandes des Verschlussabschnittes (2).

8. Chirurgiegerät nach Anspruch 7,
**dadurch gekennzeichnet, dass**
die erste Messeinrichtung (22) über eine erste Steuerung (20) zur Steuerung eines Injektionszeitpunktes und/oder Injektionsverlaufes und/oder Injektionsmenge mit der Injektionseinrichtung (10) verbunden ist.

9. Chirurgiegerät nach einem der vorhergehenden Ansprüche,
**gekennzeichnet durch**
eine zweite Messeinrichtung (23) zum Feststellen eines Temperatur- und/oder Koagulationszustandes des Verschlussabschnittes (2).

10. Chirurgiegerät nach Anspruch 9,
**dadurch gekennzeichnet, dass**
die zweite Messeinrichtung (23) über eine zweite Steuerung (20') mit einer Energiezufuhreinrichtung (40) zum Zuführen von Energie zum Verschlussabschnitt (2) zur Steuerung einer Energiemenge verbunden ist.

11. Chirurgiegerät nach einem der Ansprüche 7 - 10,
**dadurch gekennzeichnet, dass**
die erste und/oder die zweite Messeinrichtung (22, 23) als Impedanzmesseinrichtung zum Messen einer elektrischen Impedanz des Blutgefäßes (1), insbesondere im Bereich des Verschlussabschnittes (2), ausgebildet ist.

## Claims

1. Surgical instrument for sealing blood vessels (1), comprising an injection device (10) and a dosing device (20), which are configured in such a way that a predetermined amount of an adhesive (5) or an adhesive with filler can be injected into a sealing section (2) of the blood vessel (1),
**characterized by**
a clamp device (30), which has gripping elements (33-36) formed in such a way that the blood vessel (1) can be gripped and sealed at spaced-apart sealing points (3, 4) in such a way that the sealing section (2) of the blood vessel (1) that can be filled with the adhesive (5) remains between the sealing points (3, 4), and
an energy-supply device (40-42) which is intended for heating up and/or coagulating the sealing section (2) and has electrodes (41, 42) between the gripping elements (33-36) for passing a highfrequency current through the blood vessel (1), restricted to the sealing section (2).

2. Surgical instrument according to Claim 1,
**characterized in that**
the injection device (10) is connected to the clamp device (30) in such a way that the adhesive (5) can be injected into the sealing section (2).

3. Surgical instrument according to one of the preceding claims,
**characterized in that**
the injection device (10) comprises a nozzle (11) and a pressure-generating device (12), which are formed in such a way that the adhesive (5) can be injected into the blood vessel (1) while forming a sufficiently powerful jet.

4. Surgical instrument according to one of the preceding claims,
**characterized in that**
the injection device (10) comprises a needle (13) and a pressure-generating device (12), which are formed in such a way that the adhesive (5) can be injected into the blood vessel (1) by means of the needle (13).

5. Surgical instrument according to Claim 4,
**characterized in that**
the injection device (10) is formed in such a way that the needle (13) can be moved hydraulically by the pressure-generating device (12).

6. Surgical instrument according to one of the preceding claims,
**characterized by**
a cutting device (37) for severing the blood vessel (1) in the region of the sealing section (2).

7. Surgical instrument according to one of the preceding claims,
**characterized by**
a first measuring device (22) for determining a filling state of the sealing section (2).

8. Surgical instrument according to Claim 7,
**characterized in that**
the first measuring device (22) is connected to the injection device (10) via a first control unit (20) for controlling an injection time and/or injection profile and/or injection amount.

9. Surgical instrument according to one of the preceding claims,
**characterized by**
a second measuring device (23) for determining a temperature and/or coagulation state of the sealing section (2).

10. Surgical instrument according to Claim 9,
**characterized in that**
the second measuring device (23) is connected via a second control unit (20') to an energy-supply device (40) for supplying energy to the sealing section (2) to control an amount of energy.

11. Surgical instrument according to one of Claims 7 - 10,
**characterized in that**
the first and/or the second measuring device (22, 23) is formed as an impedance measuring device for measuring an electrical impedance of the blood vessel (1), in particular in the region of the sealing section (2).

## Revendications

1. Appareil chirurgical pour sceller des vaisseaux sanguins (1), comprenant un dispositif d'injection (10) et un dispositif de dosage (20), qui sont construits de telle manière qu'une quantité prédéterminée d'un adhésif (5) ou d'un adhésif avec une charge puisse être injectée dans la portion de fermeture (2) du vaisseau sanguin (1), **caractérisé par** un dispositif de pince (30), qui présente des éléments de pinçage (33-36) configurés de telle manière que le vaisseau sanguin (1) puisse être pincé et scellé en des points de fermeture (3, 4) espacés l'un de l'autre, de telle manière que la portion de fermeture (2) du vaisseau sanguin (1) à remplir avec l'adhésif (5) subsiste entre les points de fermeture (3, 4), et un dispositif d'apport d'énergie (40-42) pour le chauffage et/ou la coagulation de la portion de fermeture (2), qui présente des électrodes (41, 42) entre les éléments de pinçage (33-36) pour le passage d'un courant HF à travers le vaisseau sanguin (1), limité à la portion de fermeture (2).

2. Appareil chirurgical selon la revendication 1, **caractérisé en ce que** le dispositif d'injection (10) est relié au dispositif de pince (30), de telle manière que l'adhésif (5) puisse être injecté dans la portion de fermeture (2).

3. Appareil chirurgical selon l'une quelconque des revendications précédentes, **caractérisé en ce que** le dispositif d'injection (10) comprend un ajutage (11) et un dispositif de production de pression (12), qui sont configurés de telle manière que l'adhésif (5) puisse être injecté dans le vaisseau sanguin (1) en formant un jet suffisamment fort.

4. Appareil chirurgical selon l'une quelconque des revendications précédentes, **caractérisé en ce que** le dispositif d'injection (10) comprend une aiguille (13) et un dispositif de production de pression (12), qui sont configurés de telle manière que l'adhésif (5) puisse être injecté dans le vaisseau sanguin (1) au moyen de l'aiguille (13).

5. Appareil chirurgical selon la revendication 4, **caractérisé en ce que** le dispositif d'injection (10) est configuré de telle manière que l'aiguille (13) puisse être déplacée hydrauliquement par le dispositif de production de pression (12).

6. Appareil chirurgical selon l'une quelconque des revendications précédentes, **caractérisé par** un dispositif de coupe (37) pour sectionner le vaisseau sanguin (1) dans la région de la portion de fermeture (2).

7. Appareil chirurgical selon l'une quelconque des revendications précédentes, **caractérisé par** un premier dispositif de mesure (22) pour détecter un niveau de remplissage de la portion de fermeture (2).

8. Appareil chirurgical selon la revendication 7, **caractérisé en ce que** le premier dispositif de mesure (22) est relié au dispositif d'injection (10) par une première commande (20) pour la commande d'un instant d'injection et/ou d'une allure d'injection et/ou d'une quantité d'injection.

9. Appareil chirurgical selon l'une quelconque des revendications précédentes, **caractérisé par** un deuxième dispositif de mesure (23) pour détecter un état de température et/ou de coagulation de la portion de fermeture (2).

10. Appareil chirurgical selon la revendication 9, **caractérisé en ce que** le deuxième dispositif de mesure (23) est relié par une deuxième commande (20') à un dispositif d'apport d'énergie (40) pour apporter de l'énergie à la portion de fermeture (2) en vue de la commande d'une quantité d'énergie.

11. Appareil chirurgical selon l'une quelconque des revendications 7 à 10, **caractérisé en ce que** le premier et/ou le deuxième dispositif de mesure (22, 23) est constitué par un dispositif de mesure d'impédance pour mesurer une impédance électrique du vaisseau sanguin (1), en particulier dans la région de la portion de fermeture (2).
